# EUROPEAN PATENT APPLICATION

(11) **EP 4 173 583 A1**
(43) Date of publication of application: **03.05.2023**
(21) Application number: 22204517.1
(22) Date of filing: 28.10.2022
(51) Int. Cl.: A61B 18/14, A61B 17/32, A61B 18/16, A61M 1/00

(54) **ELECTROSURGICAL INSTRUMENT**

(30) Priority: 01.11.2021 US 202163274335 P
(71) Applicant: Gyrus Medical Limited, Cardiff CF3 0LT (GB)
(72) Inventor: DICKSON, James, Cardiff, CF3 0LT (GB); BUTTERWICK, Gillian, Cardiff, CF3 0LT (GB)
(74) Representative: Halliwell, Bethan Frances

(57) **Abstract**

The present disclosure relates to an end effector for an electrosurgical instrument, comprising an electrode assembly for delivering a radio-frequency (RF) power signal to a surgical site, the electrode assembly comprising an active electrode, a return electrode, and an insulating element in between the active electrode and the return electrode, the active electrode comprising an aperture which provides access to a suction channel extending through the insulating element to a lumen for carrying fluid from the surgical site, wherein the lumen is at least in part defined by an inner surface of the return electrode, wherein the electrode assembly is configured to conduct electrical current between the active electrode and the return electrode via a first current path through the suction channel when the RF power signal is supplied to the electrodes.

## Description

### Technical Field

The present invention relates an electrosurgical instruments. More specifically, the present invention relates to end effectors of electrosurgical instruments.

### Background to the Invention and Prior Art

Surgical instruments, including radio frequency (RF) electrosurgical instruments, have become widely used in surgical procedures where access to the surgical site is restricted to a narrow passage, for example, in minimally invasive "keyhole" surgeries.

RF electrosurgical instruments may include an active electrode which forms a distal RF tip of the instrument, and a return electrode. The distal RF tip provides tissue ablation and/or coagulation effects at a surgical site when a RF power signal is delivered to the electrodes. Known wet-field RF hand instruments for arthroscopy also often use a saline suction pathway at the distal tip during either ablating or coagulating tissue. In general terms, suction provides the following benefits:
- Cools the RF tip by drawing colder saline over the hot RF tip during use,
- Helps to remove ablated tissue debris from the surgical site,
- Helps to draw fresh tissue toward the RF tip,
- Removes bubbles to improve visibility of the surgical site, and
- Improves the formation of plasma at the tip.

When tissue is drawn into contact with the RF tip via the saline suction pathway, the tip suction holes hold the tissue against the tip during ablation via negative suction pressure. Some of the tissue can enter and temporarily block the suction hole or holes in the RF tip while the tissue is being ablated. While the suction hole is blocked, the previously listed benefits of suction are temporarily impaired.

There is thus a need to improve the suction performance of such RF electrosurgical instruments.

### Summary of the Invention

In known RF instruments, the electrical current available to create an ablation effect at the active RF tip is biased toward the outer peripheral edges of the RF tip that are closest to the electrical return electrode or electrodes. Therefore, the suction hole in the RF tip does not have a particularly high current density at the location of the hole, and therefore tissue at the hole or tissue blocked within the hole is not ablated. This is particularly of issue when the suction hole is centrally located on the RF tip. The present invention relates to an electrode assembly of a RF electrosurgical instrument that is able to clear or unclog the suction hole of tissue or surgical debris. In particular, the electrode assembly provides an increased current density at the suction hole. For example, the electrode assembly provides a current path between the active and return electrodes, through the suction hole, in order to bias current towards the suction hole. As a result, an ablation effect is biased towards the suction hole, and therefore tissue blocking or clogged in the suction hole is ablated, thereby restoring good suction.

In accordance with a first aspect of the present invention, there is provided an end effector for an electrosurgical instrument, comprising an electrode assembly for delivering a radio-frequency (RF) power signal to a surgical site, the electrode assembly comprising an active electrode, a return electrode, and an insulating element arranged between the active electrode and the return electrode, the active electrode comprising an aperture for providing access to a suction channel extending through the insulating element to a lumen configured to carry fluid from the surgical site, wherein the lumen is at least in part defined by an inner surface of the return electrode, wherein the electrode assembly is configured to conduct electrical current between the active electrode and the return electrode via a first current path through the suction channel when the RF power signal is supplied to the electrodes.

Advantageously, by conducting an electrical current through the suction channel, an ablation affect is achieved at the aperture and/or in the suction channel. Therefore, tissue that is lodged in the suction channel or blocking the channel at the aperture will be ablated, and therefore blockages will be quickly cleared during use of the end effector.

In some arrangements, the electrode assembly has a distance from the active electrode to the return electrode through the suction channel such that the electrode assembly conducts electrical current between the active electrode and the return electrode via the first current path through the suction channel when the RF power signal is supplied to the electrodes.

For example, the structure of the electrode assembly may provide a sufficiently short distance between the electrodes via the suction channel, such that an electrical current sufficient for producing a RF ablating effect at the aperture and/or in the suction channel is conducted through the suction channel.

In some arrangements, the electrode assembly is further configured to conduct electrical current between the active electrode and the return electrode via a second current path that is not through the suction channel, when the RF power signal is supplied to the electrodes.

Advantageously, by conducting current through an external current path (i.e. a current path that is not internal through the suction channel), the end effector can still perform normal ablation and/or coagulation functions at a surgical site, whilst simultaneously clearing the aperture and/or the suction channel of surgical debris.

In some arrangements, the electrode assembly is configured such that the first electrical current path conducts less current than the second electrical current path.

Advantageously, most of the total current conducted between the two electrodes is biased to the second current path. This ensures that most of the RF energy is used for performing ablation and coagulation functions at the surgical site. Although the first current path draws less current or RF energy, it can still be a sufficient amount of energy to properly ablate debris in the suction channel and provide de-clogging functions. The exact ratio of current between the first and the second current paths may be dependent on the use case for the present invention, and can be determined using experimentation of finite element analysis (FEA).

In some arrangements, the active electrode is distanced from the return electrode by a first distance via the first current path, and the active electrode is distanced from the return electrode by a second distance via the second current path, wherein the first distance is greater than the second distance such that the first electrical current path conducts less current than the second electrical current path.

This arrangement illustrates an example way of biasing most of the total current between the electrodes to the second current path. In particular this can be achieved by configuring the physical construction of the electrode assembly so that the first current path is longer than the second current path. This will mean that less current is drawn through the first current path, therefore biasing most of the total current to the second current path. It will be appreciated that the shape and size of the electrodes and the insulating element can be varied in any number of ways to achieve the appropriate distances. The ideal structure for the electrode assembly, and the ideal distances, may be dependent on the specific use case, and can be determined using experimentation and/or finite element analysis techniques.

In some arrangements, the aperture is located substantially in the centre of the active electrode.

When the aperture is in the centre of the active electrode, it is more susceptible to clogging because it is maximally distanced from the peripheral edge of the active electrode. Therefore, the invention is particularly advantageous for unclogging apertures and suction channels where the aperture is located in the centre of the active electrode.

In some arrangements, the end effector further comprises a rotatable shaver blade that is partially and concentrically surrounded by the return electrode.

Advantageously, the end effector can achieve mechanical cutting/shaving functions in addition to RF ablation/coagulation functions. The return electrode may be considered as a stator, and together with the rotatable shaver blade form a rotary shaver arrangement.

In some arrangements, the return electrode comprises cutting teeth and a shaving window framed by the cutting teeth, wherein the shaving window is on an opposite side to the active electrode.

Advantageously, the techniques of the present invention can be used in dual (or opposite) sided RF shaver devices or end effectors. In other examples, the mechanical shaving window can be on the same side as the active electrode to define a single sided RF shaver device, as defined in UK patent publication GB2582318. As such, the techniques of the present invention could also be applied in single sided RF devices.

In some arrangements, the rotatable shaver blade is rotatable to a position in which the inner surface of the return electrode is exposed to the suction channel.

Advantageously, the end effector can be operated in a mode in which the inner surface is exposed to the suction channel, thereby providing the first current path between the active electrode and the inner surface of the return electrode.

The rotatable shaver blade may be made from an electrically conductive material or a nonconductive material.

Advantageously, the rotatable shaver blade can be made of a variety of materials. Moreover, if the rotatable shaver blade is electrically conductive, the rotatable shaver blade does not need to be in a position which exposes the inner surface of the return electrode to the suction channel in order to provide the first current path, since the first current path can conduct through the rotatable shaver blade.

In some arrangements, the second current path is from a peripheral edge of the active electrode over a peripheral edge of the insulating element to an outer surface of the return electrode.

Advantageously, a high current density is supplied to the peripheral edge of the active electrode, which is better for providing ablation and/or coagulation effects at a surgical site.

In some arrangements, the end effector further comprises a retainer arranged to hold the active electrode in place on the insulating element.

Advantageously, the active electrode can be attached held securely in place to the insulator during use.

In some arrangements, the active electrode comprises one or more protrusions.

Advantageously, the protrusions may focus or concentrate the electric field generated by the second current path at the locations of the protrusions. The protrusions also serve to create a small separation between the planar top surface of the active electrode and the tissue to be treated at the surgical site. This allows conductive fluid to circulate over the planar surface, and avoids overheating of the electrode or the tissue.

In some arrangements, the active electrode is formed from a metal, and preferably wherein the metal is any one of copper, stainless steel, tungsten or an alloy of tungsten and platinum.

In some arrangements, the return electrode is formed from a metal, and preferably wherein the metal is any one of copper, stainless steel, tungsten or an alloy of tungsten and platinum.

In some arrangements, the insulating element is formed of a ceramic or a polymer.

In some arrangements, the lumen is connectable to a suction tube for connecting to a suction source.

Advantageously, suction can be provided to the aperture from a suction source to transport surgical debris and tissue away from the surgical site.

In some arrangements, the active electrode and the return electrode are connectable to a RF power source.

Advantageously, the RF power signal can be supplied to the electrodes from an external power source to perform the ablation and/or coagulation functions described herein.

In some arrangements, the return electrode forms an outer shaft of the end effector.

In a second aspect of the present invention, there is provided an electrosurgical instrument, comprising: a hand-piece; one or more user-operable buttons on the handpiece for operably controlling the instrument, and an operative shaft, having RF electrical connections, and drive componentry for an end effector, the electrosurgical instrument further comprising an end effector according to the first aspect, the active electrode and the return electrode being connected to the RF electrical connections.

In a third aspect of the present invention, there is provided an electrosurgical system, comprising: an RF electrosurgical generator; a suction source; and an electrosurgical instrument according to the second aspect, the arrangement being such that in use the RF electrosurgical generator supplies an RF coagulation or ablation signal via the RF electrical connections to the active electrode and the return electrode.

### Brief Description of the Drawings

Embodiments of the invention will now be further described by way of example only and with reference to the accompanying drawings, wherein like reference numerals refer to like parts, and wherein:
Figure 1 shows an example of an electrosurgical instrument system comprising an RF electrosurgical instrument according to the present invention;
Figure 2 shows a perspective view of a distal end portion of a dual (or opposite) sided RF electrosurgical instrument;
Figure 3A shows a cross-sectional perspective diagram of the RF shaver of Figure 2 which achieves an increased current density and an ablation effect in/at the suction channel;
Figure 3B shows a cross-sectional diagram of the RF shaver of Figure 3A;
Figure 4A shows a cross-sectional perspective diagram of a RF device which may not achieve an ablation effect in/at the suction channel;
Figure 4B shows a cross-sectional diagram of the RF device of Figure 4A;
Figures 5A-5C illustrate the RF device of Figures 4A-4B in use when tissue is introduced to the suction channel; and
Figures 6A-6D illustrate the dual (or opposite) sided RF shaver of Figures 3A-3B in use when tissue is introduced to the suction channel.

### Detailed Description of the Embodiments

Embodiments of the present invention relate to an electrode assembly of a RF electrosurgical instrument (e.g. a RF shaver device). The electrode assembly is located at a distal end portion of the instrument. The electrode assembly includes an active electrode and a return electrode separated physically by an insulating element. A suction hole is included in the active electrode for removing tissue and debris from the surgical site. The suction hole leads to a suction lumen via a suction channel, for transporting the tissue and debris to a suction source. Current will conduct between the active and return electrodes over the exterior of the instrument when a RF power signal is supplied to the electrodes. This provides external tissue ablation and coagulation functions at the surgical site, in accordance with known RF electrosurgical instruments. Further, in a RF operating mode of the instrument, an interior surface of the return electrode is exposed to the suction channel. The distance between the active electrode and the interior surface of the return electrode is short enough such that some current will also conduct internally between the active and return electrodes through the suction hole. Consequently, there is an increased current density at and/or in the suction hole which results in an (internal) ablation effect at and/or in the suction hole. Tissue that is blocking the suction hole or clogging the suction channel will therefore be ablated, clearing the suction pathway of tissue.

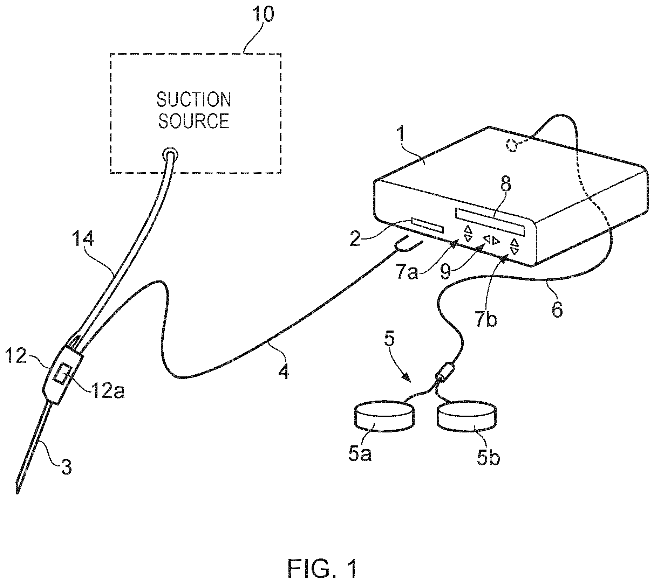

Figure 1 shows an electrosurgical apparatus including an electrosurgical generator 1 having an output socket 2 that provides a radio frequency (RF) output (e.g. a RF power signal), via a connection cord 4, to an electrosurgical instrument 3. The instrument 3 has a suction tube 14 which is connected to a suction source 10. Activation of the generator 1 may be performed from the instrument 3 via a handswitch 12a on the handpiece 12 of the instrument 3, or by means of a footswitch unit 5 connected separately to the rear of the generator 1 by a footswitch connection cord 6. In the illustrated embodiment, the footswitch unit 5 has two footswitches 5a and 5b for selecting a coagulation mode, or a cutting or vaporisation (ablation) mode of the generator 1 respectively. The generator front panel has push buttons 7a and 7b for respectively setting ablation (cutting) or coagulation power levels, which are indicated in a display 8. Push buttons 9 are provided as an alternative means for selection between the ablation (cutting) and coagulation modes.

The electrosurgical instrument 3 can be a dual sided (or an opposite sided) RF shaver device. In particular, the main RF componentry and the main mechanical shaving/cutting componentry of the instrument 3 can be provided on opposite sides of a distal end portion of the instrument 3. In such cases, any of the switching means described above may be provided for selecting the mechanical shaving mode. The structure of the distal end of the instrument 3 is described in more detail below.

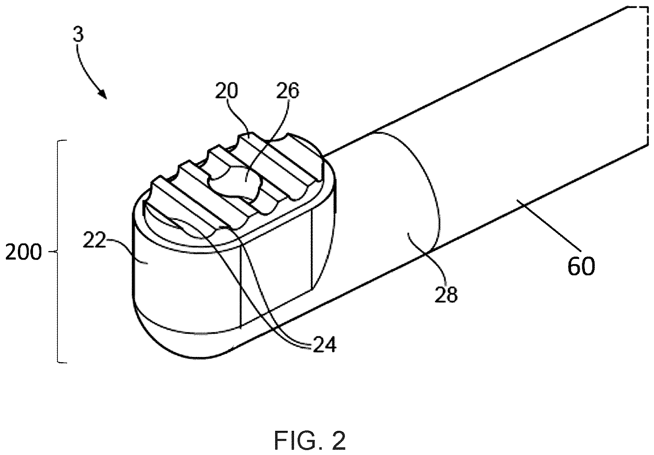

Figure 2 shows a perspective view of the distal end portion of the electrosurgical instrument 3 according to an embodiment. In particular, an RF side of the distal end portion of the electrosurgical instrument 3 is shown. The RF side of the electrosurgical instrument 3 includes an electrode assembly 200 having an active electrode 20 for tissue treatment (i.e. the "active tip"), an insulating element 22, and a return electrode 28. The active electrode 20 is received in the insulator 22. The insulator 22 is provided in between the active electrode 20 and the return electrode 28 to physically separate the active electrode 20 from the return electrode 29. The active electrode 20 and the return electrode 28 receive the RF power signal from the generator 1 (not shown). As a result, the active electrode 20 will produce an electric field in response to the RF power signal. The electric field is used to treat tissue at a surgical site in the proximity of the active tip 20. As illustrated in Figure 2, the active tip 20 may also be provided with projections or protrusions 24 to concentrate the electric field at the locations of the projections 24. The projections 24 also serve to create a small separation between the planar top surface of the active electrode 20 and the tissue to be treated at the surgical site. This allows conductive fluid to circulate over the planar surface, and avoids overheating of the electrode or the tissue.

The active tip 20 of the instrument is provided with a primary suction aperture 26, which is the opening to a primary suction or fluid channel (not shown). The primary suction channel extends to a lumen (not shown). The lumen may extend through the return electrode and an outer shaft 60 of the instrument, to the suction tube 14. The lumen therefore connects the suction aperture 26 to the suction pump 10 (see Figure 1) to transport fluids from the active tip 20 to the pump 10. The lumen may also include means (e.g. wires) for electrically connecting the active electrode 20 to the generator 1. The return electrode 28 may be connected to the generator 1 via the outer shaft 60 of the instrument 3. In particular, the return electrode 28 may be electrically connected to the outer shaft 60 of the instrument 3 and the outer shaft 60 may be electrically conductive. Alternatively, the shaft 60 may form the same component as the return electrode 28, and therefore the shaft may be considered as the return electrode. In any case, the RF power signal can be delivered from the generator 1 to the active 20 and return 28 electrodes, via the cord 4.

As discussed above, the electrosurgical instrument 3 may be a dual-sided instrument with a mechanical shaver. The mechanical shaver/cutting side of the electrosurgical instrument 3 is not shown in Figure 2, however, this may be in the form of an internal shaver blade that rotates within the lumen to thereby cut tissue through a shaving window arranged on the opposite side of the active electrode tip 20, as will be described in more detail below. In use, the shaver blade will cut tissue that is presented adjacent to the cutting window.

The active electrode tip 20 is formed of an electrically conductive material. The electrically conductive material may be any material suitable for forming an active electrode tip 20, for example, a metal such as copper or a stainless steel, tungsten or an alloy of tungsten and platinum. The insulator 22 can be a ceramic insulator. Alternatively, the insulator 22 can be made from a polymer. The insulator 22 can otherwise be any other suitable material for providing an insulation from electrical contact. The return electrode 28 is also formed of an electrically conductive material. The electrically conductive material of the return electrode 28 may be any material suitable for forming a return electrode, for example, a metal such as copper or a stainless steel, tungsten or an alloy of tungsten and platinum. The electrically conductive material of the outer shaft 60 may also be for example, a metal such as copper or a stainless steel, tungsten or an alloy of tungsten and platinum.

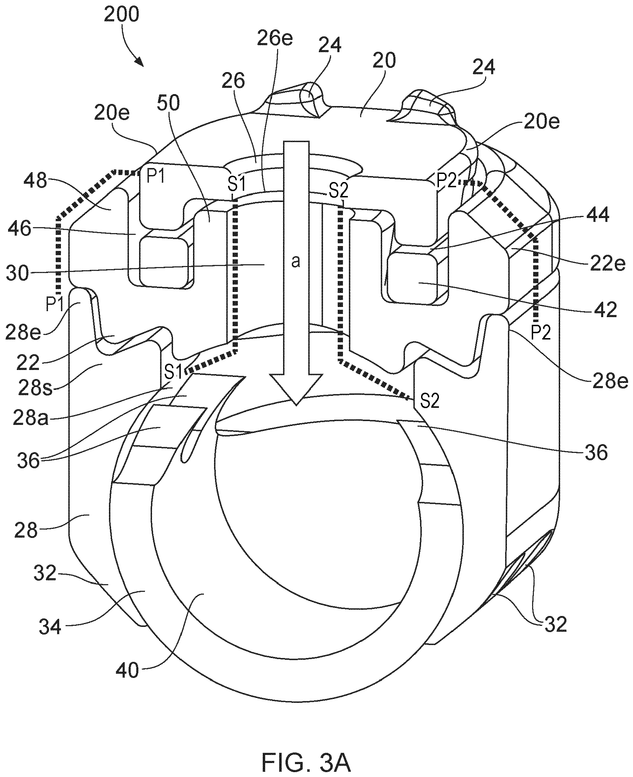

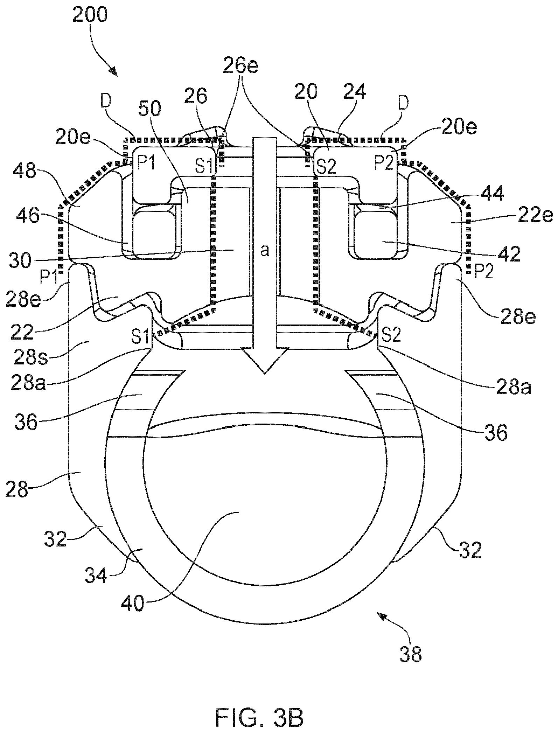

Figures 3A and 3B shows cross sections of the distal end portion of the RF electrosurgical instrument 3. Figure 3A shows a perspective cross section of the distal end portion, and Figure 3B shows a regular cross section of the distal end portion. Figures 3A and 3B show both the RF side of the electrosurgical instrument 3 (top side) and the mechanical shaver/cutting side of the electrosurgical instrument 3 (bottom side).

Figures 3A-3B show the active tip 20 having the projections 24. The active tip 20 may be held in place in the insulator 22 by a retainer 42. Where the retainer 42 and the active tip 20 meet is referred to as the mating portion 44. Alternatively, the active tip 20 and the retainer 42 may be formed as a single component (a "one-piece tip"), without the mating portion 44. The insulating material 22 comprises a recess 46 where the retainer 42 is positioned. The recess 46 is defined by outer 48 and inner 50 lips. On top of the retainer 42, the active tip 20 is held in place such that the active tip 20 extends above the outer lips 48 of the insulating material 22. In the case of a one-piece tip, the active tip 20 is positioned directly into the recess 46 of the insulting material 22.

Figures 3A-3B show the primary suction aperture 26, which is the opening to a primary suction/fluid channel 30 extending to a lumen 40. The lumen 40 provides a suction passage to the suction source 10 as described above. The arrow a indicates the saline suction path during RF use of the instrument 3 (i.e. through the primary suction channel 30). The primary suction channel 30 extends from the surface of the active tip 20 through the active tip 20 and the insulating material 22 to the lumen 40. The opening of the primary suction channel 26 is located on the surface of the active tip 20 ("the primary suction aperture" 26). The primary suction aperture 26 may be located in the centre of the active tip 20, or offset to one side. The primary suction channel 30 runs between the inner lip 50 from the primary suction aperture 26 directly down to the lumen 40. The lumen 40 is connected to the suction source 10 via suction tubes 14 (as shown in Figure 1).

Figures 3A-3B also show the return electrode 28. The return electrode 28 is connected to the bottom of the insulating component 22 such that the insulating component 22 insulates the active tip 20 from the stator 28. In particular, the insulating component 22 insulates the active tip 20 from direct electrical contact with the return electrode 28. Current may still indirectly conduct between the active 20 and return 28 electrodes as described below. The insulating material 22 may be, for example, a ceramic material such as alumina, zirconia toughened alumina (ZTA), yttria stabilized zirconia (YTZP) or the like.

The return electrode 28 has cutting teeth 32. The instrument 3 also comprises an inner shaver blade 34 having cutting teeth 36. The return electrode 28 and the inner shaft 34 are concentrically arranged such that the cutting teeth 32, 36 frame a cutting window 38 formed in a bottom side of the return electrode 28. As such, the distal end of the instrument 3 also comprises a rotary shaver formed of the inner shaver blade 34 and the return electrode 28, in particular the teeth 32 of the return electrode 28. The return electrode 28 can be considered as a stator of the rotary shaver. When the rotary shaver component (i.e. the inner and the outer blades) is in use, the inner shaver blade 34 rotates such that the inner 36 and the outer 32 teeth cut tissue. In Figures 3A-3B, the shaver component is not in use, and the cutting window 38 is closed by an outer surface of the inner shaver blade 34. As such, Figures 3A-3B may show the instrument 3 in a RF mode in which the RF power signal is supplied to the electrodes 20 and 28 to provide RF functionality, whilst the mechanical shaving functionalities remain off. To perform mechanical shaving, the inner shaver 34 may receive power from the generator 1 to rotate, for example via the cord 4 or otherwise. The interior surfaces of the return electrode 28 and the inner shaver blade 34 defines a starting portion of the lumen 40 which carries fluid from the active tip 20 to the suction source 10. The return electrode 28 is made of an electrically conductive material, for example a metal such as copper, stainless steel, tungsten or an alloy of tungsten and platinum.

In some examples, the inner blade 36 can be made from an insulated material (e.g. insulated steel, or fully ceramic). Alternatively, the inner blade 34 can be an electrically conductive material, e.g. a metal, such as copper, stainless steel, tungsten or an alloy of tungsten and platinum.

As discussed above, the return electrode 28 and the active electrode 20 receive the RF power signal from the generator 1, for example via the cord 4. The return electrode 28 is electrically connected to output 2 of the generator 1 via the outer shaft 60 (not shown in Figure 3) to receive the RF power signal from the generator 1. The active tip 22 is also connected to the generator 1 via the cord 4 to receive the RF power signal from the generator 1. The active tip 22 may be connected to the cord 4 via electrical wiring.

During RF operation of the instrument 3, the RF power signal is supplied to the electrodes 20/28 from the generator 1. Although the active electrode 20 is physically distanced from the return electrode 28, electrical current will conduct between the electrodes 20/28 due to the RF power signal. Consequently, an electric field is generated at the active tip 20, which can be used to perform RF functions at a surgical site, such as tissue ablation and/or coagulation. Simultaneously, the instrument 3 will perform suction functions. In particular, the suction source 10 provides suction at the suction aperture 26 via the suction tube 14, the lumen 40 and the suction channel 30. The suction is used to remove ablated tissue debris from the surgical site, draw fresh tissue towards the RF tip, remove bubbles from the surgical site to improve visibility, enhance plasma formation at the active tip 20 and cool the RF tip by drawing cooler saline over the active tip 20. However, soft tissue that has not been sufficiently ablated can enter and temporarily block the aperture 26 and/or the suction channel 30. This can impair the benefits of the suction described above.

Advantageously, the instrument 3 has the ability to ablate tissue that may be blocking or clogging the aperture 26 and/or the suction channel 30. The lines P1-P1 and P2-P2 in Figures 3A-3B illustrate primary current paths between the active electrode 20 and the return electrode 28. The lines S1-S1 and S2-S2 illustrate secondary current paths between the active electrode 20 and the return electrode 28.

The primary current paths P1-P1 and P2-P2 are external current paths that conduct or track over the outside or exterior of the distal end portion. For example, the primary current paths may be considered as traversing or conducting between a peripheral edge 20e of the active tip 20 and an exterior surface 28s (more particularly an upper edge 28e) of the return electrode 28, over a peripheral edge 22e of the insulating element 22. The primary current paths bias part of the RF energy from the generator 1 to the peripheral edge 20e of the active tip 20. As such, the primary current paths are responsible for generating the electric field that provides the RF functionalities at the surgical site, such as (external) tissue ablation and/or coagulation as described above. In particular, in operation, there is a high current density at the peripheral edge 20e of the active tip 20 for performing RF functions at the surgical site.

The secondary current paths S1-S1 and S2-S2 are internal current paths that conduct or track through the interior of the distal end portion. For example, the secondary current paths may be considered as traversing between the aperture 26 of the active electrode 20 (e.g. an inner rim 26e of the aperture 26) to an inner surface 28a of the return electrode 28. In Figures 3A-3B, the shaving window 38 is in a closed position in the RF operating mode of the instrument 3. Therefore, the shaver blade 34 is in a rotational position that exposes the inner surface 28a of the return electrode 28 to the suction channel 30. The secondary current paths may not generate an external "clinical" effect that contributes to tissue coagulation and/or ablation at the surgical site. However, the secondary current paths increase the current density at the aperture 26 and in the suction channel 30. As such, the secondary current paths can ablate tissue that is blocking the aperture 26 or clogging the suction channel 30, thereby reducing clogging and blocking of the suction pathway between the aperture 26 and the lumen 40. As shown by the region D in Figure 3B, the primary and secondary current pathways may result in a current density at the peripheral edges 20e of the active tip 20, which extends towards and into the aperture 26 and thus into the suction channel 30.

The current paths S1-S1 and S2-S2 conduct current because the structure of the electrode assembly 200 ensures that the tracking distances of those paths are short enough to conduct current when the electrodes receive the RF power signal. In particular, the electrode assembly 200 may be structured to ensure that the distance between the aperture 26 (more particularly the inner rim 26e) and the inner surface 28a of the return electrode 28 via the suction channel 30 is short enough to allow a current to conduct therebetween, e.g. via the paths S1-S1 and S2-S2.

It is preferred that the tracking distances or lengths of the secondary current paths S1-S1 and S2-S2 are greater than the tracking distances or lengths of the primary current paths P1-P1 and P2-P2. More particularly, it may be preferred that the distance between the aperture 26 (e.g. the inner rim 26e of the aperture 26) and the inner surface 28a of the return electrode 28 via the channel 30 as indicated by the lines S1-S1 or S2-S2, is greater than the distance between the peripheral edge 20e of the active tip 20 and the upper edge 28e of the return electrode 28 over the exterior of the distal end portion as indicated by the lines P1-P1 or P2-P2. This may ensure that more current (or more RF energy) is biased towards the peripheral edge 20e of the active tip 20 than to the suction channel 30. As such, instrument 3 can maintain good ablation and coagulation performance at the peripheral edge 20e. Otherwise, if the tracking distance of the secondary current paths is too short (e.g. shorter than the tracking distance of the primary current paths) then this may negatively impact the ablation and coagulation performance at the peripheral edge 20e. In particular, the instrument 3 may not properly performing the intended external RF functions at the surgical site. However, it will be appreciated that the longer the secondary current paths are made, the smaller the unclogging ablation effect at the aperture 26 or the suction channel 30. As such, the tracking distances of the primary and the secondary current paths are preferably balanced to achieve effective declogging effects at the aperture 26 and in the suction channel 30, whilst maintaining good external RF performance.

The exact ratio or balance of the tracking distances may depend on the intended application of the instrument 3, the type of tissue to be expected, the size of the aperture 26 and the amount of RF plasma or energy desired for achieving RF functionality. Moreover, the ratio or balance of the tracking distances may depend on the maximum RF power outputted by the electrode assembly 200. In some examples, the tracking distances or lengths of the primary current paths P1-P1 and P2-P2 are 1.3mm to 1.5mm. The tracking distances or lengths of the secondary current paths S1-S1 and S2-S2 are 1.6mm to 2.5mm. Since the tracking distance for the primary current paths will be shorter than the tracking distance for the secondary current paths, this may ensure that good external RF ablation performance is maintained whilst diverting enough current to the aperture 26 to perform de-clogging functions at the aperture and the channel 30. These ranges for the tracking distances may be suitable for an electrode assembly with a maximum RF power output in the range of 200W to 380W. The exact values of the tracking distances within those ranges may depend on the expected tissue type, the size of the aperture 26, and the maximum RF power output and/or voltages of the electrode assembly. In one specific example, the maximum RF power output can be approximately 380W, the tracking distances or lengths of the primary current paths P1-P1 and P2-P2 can be approximately 1.4mm, and the tracking distances or lengths of the secondary current paths S1-S1 and S2-S2 can be approximately 1.9mm. Alternatively, the tracking distance of the secondary current paths is approximately 1.8mm or 2.1mm. It will be appreciated that the above example ranges and values are exemplary, and any other ranges and values for the tracking distances are possible depending on the device construction and power output capabilities. In general, the precise tracking distances for a specific implementation of the invention may be determined by experimentation and/or finite element analysis (FEA) modelling.

The tracking distances of the primary and the secondary current paths can be changed by varying the structure of the electrode assembly 200. Preferably, the geometry of the suction aperture 26 and/or the height of the active tip 20 relative to the return electrode 28 is varied in order to vary the tracking distances. In one example, the distance between the inner surface 28a of the return electrode 28 and the channel 30 is varied in order to lengthen or shorten the tracking distance of the secondary current paths. This allows for the design and manufacture of electrode assemblies with different internal tracking distances, without substantially changing the overall structure of the electrode assembly. In some examples, the structure of the insulating element 22 may be varied to achieve different tracking distances. In one example, the length of the channel 30 can be varied to vary the length of the secondary current paths. Additionally or alternatively, the thickness of the lip 48 can be varied to vary the length of the primary current paths.

For the sake of simplicity, the current paths P1-P1, P2-P2, S1-S1 and S2-S2 between the active tip 20 and the return electrode 28 have been represented in 2D. In particular, the current paths are shown as traversing between single points on the active electrode 20 and the return electrode 28. It will be appreciated that, in practice, current will flow between the active and the return electrodes in a 3D manner. In particular, current will traverse between an area or areas (e.g. many points and locations) on the active electrode 20 to an area or areas (e.g. many points and locations) on the return electrode 28. As such, in operation, a current density will flow between the electrodes.

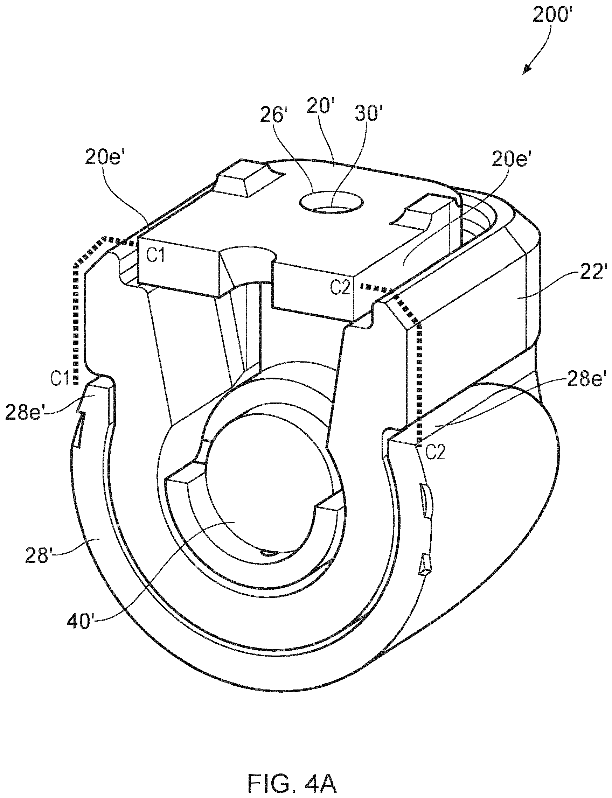

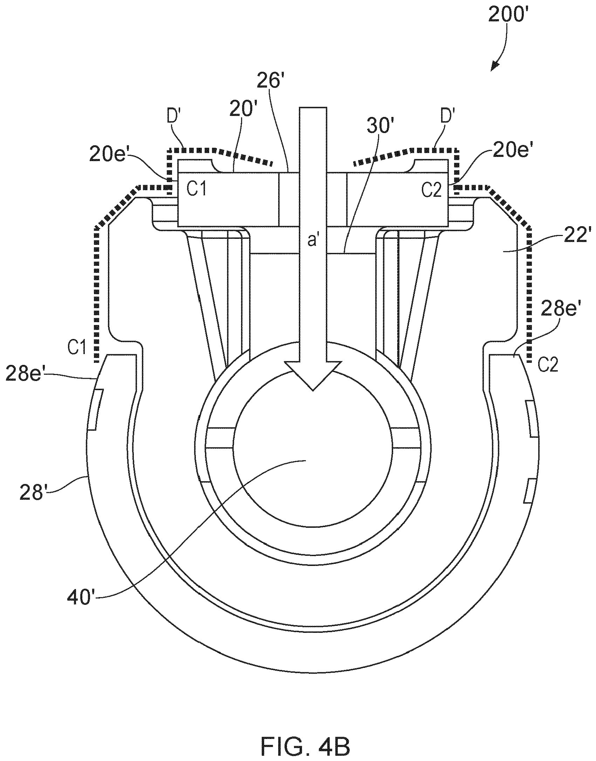

For comparison, Figures 4A-4B illustrate cross sectional views of an electrode assembly 200' that may not achieve the advantages of the present invention. Figure 4A shows a perspective cross section of the electrode assembly 200', and Figure 4B shows a regular cross section of the electrode assembly 200'. The electrode assembly 200' includes an active electrode 20' and a return electrode 28' separated by an insulating element 22'. The active tip 20' also comprises a suction aperture 26' which provides access to a suction lumen 40' via a suction channel 30' for transporting fluid and/or surgical debris away from a surgical site. The arrow a' shows the suction path through the aperture 26' and channel 30' to the lumen 40'.

During operation, a RF power signal may be supplied to the active 20' and return 28' electrodes. A current will conduct between the active electrode 20' and the return electrode 28' via current paths indicated by the lines c1-c1 and c2-c2. The current paths c1-c1 and c2-c2 extend between a peripheral edge 20e' of the active tip 20' and an upper edge 28e' of the return electrode 28'. Consequently, there will be a high current density at the peripheral edge 28' and on the top surface of the active tip 20', as indicated by the lines D'. The high current density region D' will provide the RF ablation and/or coagulation functionalities at the surgical site. However, unlike the electrode assembly 200, there is not a current path through the suction channel 30'. As such, the electrode assembly 200' is susceptible to clogging in the suction channel 30'. In particular, it has been observed through finite element analysis (FEA) and experimentation that once the suction channel 30' or aperture 26' is blocked with tissue, the concentration of plasma remains at the peripheral edge 20e' and so the RF ablation effect is still concentrated at the edge 20e'. Therefore, the electrode assembly 200' may not have the ability to unblock or unclog the aperture 26' or the suction channel 30'.

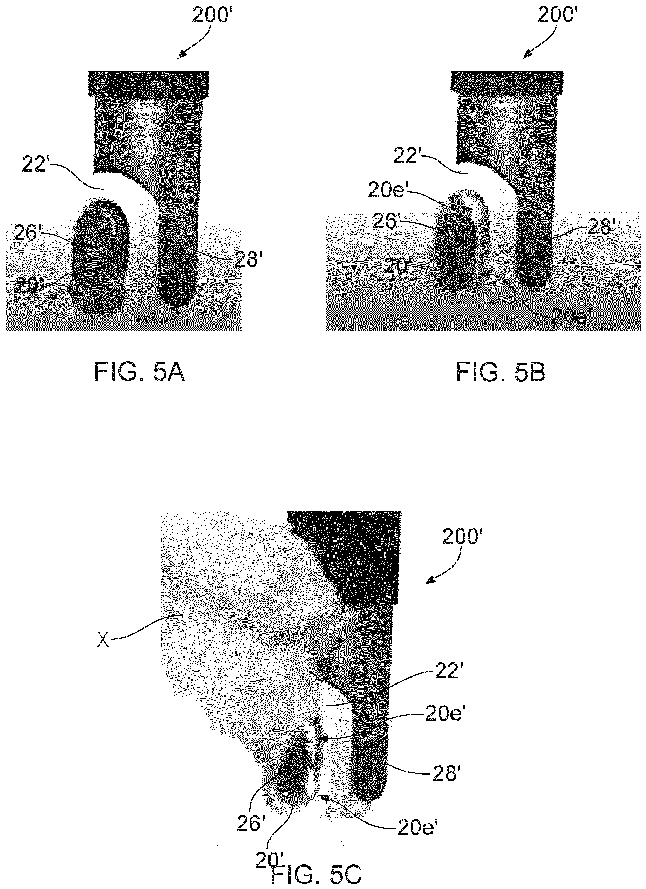

Figures 5A-5C show the electrode assembly 200' described with reference to Figures 4A-B in use. Figure 5A illustrates the active tip 20', the return electrode 28', the insulating element 22' and the aperture 26' that leads to the suction channel (not shown). Figure 5B shows the electrode assembly 200' when a RF power signal is supplied to the electrodes 20' and 28'. As shown, the active tip 20'"fires up" (e.g. a strong electric field or current density is generated) at the peripheral edges 20e' as a result of the current traversing the current paths c1-c1 and c2-c2. Figure 5C shows the electrode assembly 200' when tissue X is then introduced to the aperture 26'. As shown, the aperture 26' location does not fire up, and only the peripheral edge 28e' fires up.

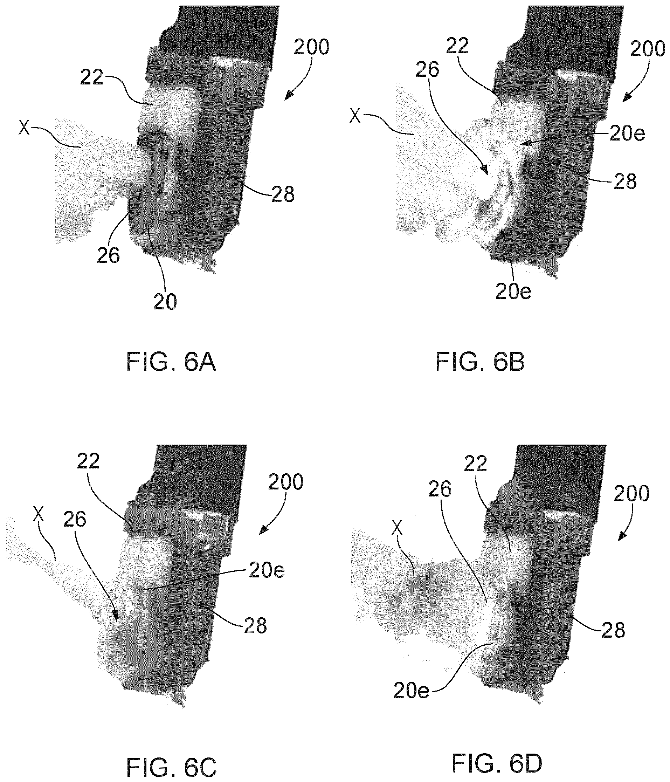

Figures 6A-6D show the electrode assembly 200 of the present invention in use. Figure 6A shows the electrode assembly 200 including the active tip 20, the return electrode 28, the insulating element 22 and the aperture 26 which leads to the suction lumen via the suction channel (not shown). Figure 6A also shows the tissue X is introduced to the aperture 26, but before the RF functionality is enabled (e.g. before the electrodes 20 and 28 are supplied with the RF power signal). Figure 6B shows the electrode assembly 200 when the RF functionality is enabled (e.g. the electrodes 20 and 28 are supplied with the RF power signal). As shown, the active tip 20 fires up at the peripheral edges 20e, e.g. as a result of current traversing the primary current paths p1-p1 and p2-p2. The active tip 20 also fires up at the aperture 26, e.g. as a result of current traversing the secondary current paths s1-s1 and s2-s2. As such, plasma is generated at the aperture 26 as the tissue X is ablated. Figures 6C-6D show how the aperture 26 may continue to fire up in addition to the peripheral edge 28e to further ablate the tissue X blocking the aperture. As such, the electrode assembly 200' is able to unblock or unclog the aperture 26 and/or the suction channel 30 (not shown).

It will be appreciated that although the instrument 3 is generally described having a dual (or opposite) sided distal end portion, the techniques of the present disclosure may be applied to RF shaving instruments that are single sided. For example, the techniques of the present disclosure can be applied to RF shaving instruments where the mechanical shaving window 38 is on the same side of the distal end portion as the active tip 20.

Similarly, it will be appreciated that the techniques of the present disclosure can be implemented in RF electrosurgical instruments that have RF functionality but do not have mechanical shaving capabilities (e.g. in single sided RF-only devices). For example, the mechanical shaving componentry of the instrument 3, (e.g. at least the shaver blade 34 and optionally the teeth 32) may be omitted. In one example, the inner shaving blade 34, the teeth 32 and the shaving window 38 can be omitted. Instead, the return electrode 28 may extend across the bottom side (previously the mechanical shaving side) of the assembly 200 to close the bottom side of the assembly and provide the suction lumen 40.

It is described above that during mechanical shaving modes of operation of the assembly 200, the inner shaving blade 34 rotates within the assembly such that the teeth 32 and 36 cut tissue. It will be appreciated that, in other examples, the shaving blade 34 can be configured to otherwise move in a reciprocating motion within the assembly, such that the teeth 32 and 36 cut tissue.

## Claims

1. An end effector for an electrosurgical instrument, comprising:
an electrode assembly for delivering a radio-frequency (RF) power signal to a surgical site, the electrode assembly comprising an active electrode, a return electrode, and an insulating element arranged between the active electrode and the return electrode, the active electrode comprising an aperture for providing access to a suction channel extending through the insulating element to a lumen configured to carry fluid from the surgical site, wherein the lumen is at least in part defined by an inner surface of the return electrode,
wherein the electrode assembly is configured to conduct electrical current between the active electrode and the return electrode via a first current path through the suction channel when the RF power signal is supplied to the electrodes.

2. The end effector of claim 1, wherein the electrode assembly has a distance from the active electrode to the return electrode through the suction channel such that the electrode assembly conducts electrical current between the active electrode and the return electrode via the first current path through the suction channel when the RF power signal is supplied to the electrodes.

3. The end effector of claim 1 or claim 2, wherein the electrode assembly is further configured to conduct electrical current between the active electrode and the return electrode via a second current path that is not through the suction channel, when the RF power signal is supplied to the electrodes.

4. The end effector of claim 3, wherein the electrode assembly is configured such that the first electrical current path conducts less current than the second electrical current path.

5. The end effector of claim 4, wherein the active electrode is distanced from the return electrode by a first distance via the first current path, and the active electrode is distanced from the return electrode by a second distance via the second current path,
wherein the first distance is greater than the second distance such that the first electrical current path conducts less current than the second electrical current path.

6. The end effector of any preceding claim, wherein the aperture is located substantially in the centre of the active electrode.

7. The end effector of any preceding claim, further comprising a rotatable shaver blade partially and concentrically surrounded by the return electrode.

8. The end effector of claim 7, wherein the return electrode comprises cutting teeth and a shaving window framed by the cutting teeth, wherein the shaving window is on an opposite side to the active electrode.

9. The end effector of any of claims 7 or 8, wherein the rotatable shaver blade is rotatable to a position in which the inner surface of the return electrode is exposed to the suction channel.

10. The end effector of any preceding claim, wherein the second current path is from a peripheral edge of the active electrode over a peripheral edge of the insulating element to an outer surface of the return electrode.

11. The end effector of any preceding claim, further comprising a retainer arranged to hold the active electrode in place on the insulating element.

12. The end effector of any preceding claim, wherein any one or more of the following applies:
the active electrode comprises one or more protrusions;
the active electrode is formed from a metal, and preferably wherein the metal is any one of copper, stainless steel, tungsten or an alloy of tungsten and platinum;
the return electrode is formed from a metal, and preferably wherein the metal is any one of copper, stainless steel, tungsten or an alloy of tungsten and platinum; and
the insulating element is formed of a ceramic or a polymer.

13. The end effector of any preceding claim, wherein any one or more of the following applies:
the lumen is connectable to a suction tube for connecting to a suction source;
the active electrode and the return electrode are connectable to a RF power source; and
the return electrode forms an outer shaft of the end effector.

14. An electrosurgical instrument, comprising:
a hand-piece;
one or more user-operable buttons on the handpiece for operably controlling the instrument, and
an operative shaft, having RF electrical connections, and drive componentry for an end effector, the electrosurgical instrument further comprising an end effector according to any of the preceding claims, the active electrode and the return electrode being connected to the RF electrical connections.

15. An electrosurgical system, comprising:
an RF electrosurgical generator;
a suction source; and
an electrosurgical instrument according to claim 14, the arrangement being such that in use the RF electrosurgical generator supplies an RF coagulation or ablation signal via the RF electrical connections to the active electrode and the return electrode.
